# EUROPEAN PATENT SPECIFICATION

(11) **EP 1 236 655 B1**
(45) Date of publication and mention of the grant of the patent: **16.04.2008**
(21) Application number: 02251212.3
(22) Date of filing: 22.02.2002
(51) Int. Cl.: B65D 81/03

(54) **Protective sheet**
Schutzfolie
Film de protection

(30) Priority: 28.02.2001 GB 0104917
(43) Date of publication of application: 04.09.2002
(73) Proprietor: Baker, Christopher, 888 Ecclesall Road Sheffield S11 8TP (GB)
(72) Inventor: Baker, Christopher Michael, Stockport, Cheshire, SK7 3NE (GB)
(74) Representative: Walsh, David Patrick

(56) References cited:
- GB-A- 2 347 662
- US-A- 3 745 998

## Description

The present invention relates to a protective device, in particular, a protective sheet for use in packaging and other applications where a protective covering would be beneficial for fragile items or items which could otherwise be damaged.

The most conventional and widely used method of protective packaging is commonly termed the "jiffy bag". This type of bag has a cushioning layer inside a conventional envelope. However, such bags do not provide a high level of resistance to breakage and are susceptible to twisting and bending forces which if applied may cause damage to objects placed inside the bag.

EP 0460942 describes a packaging sheet which comprises a sealed plastic envelope containing beads of blown polystyrene. The sheet has perforations so that when the bag is placed around an object to be packaged and pressure is applied, air is forced out of the sheet and the bead filling contracts and forms a fairly rigid protective sheet.

However, the product described in EP 0460942 is cumbersome to use and requires a sealed crate or box to compress the sheet after the packaging sheet has been located around the object. Such compression could damage the object around which the sheet is placed.

US 3,745,998, fig. 13, discloses a vacuum formed support structure convertible between a collapsed mode and rigidified support mode, according to the preamble of claim 1.

According to a first aspect of the present invention there is provided a protective device comprising a fluid sealable envelope of a non-porous material, the said envelope having a plurality of resiliently deformable members located therein, the envelope being designed to have a fluid evacuated state in which the said plurality of resiliently deformable members is operable to form into an at least partially rigid state within the envelope and sealing means to retain the envelope in the said fluid evacuated state characterised in that the device includes a porous inner lining designed to accommodate an object to be protected and wherein the lining comprises a porous inner envelope which is in fluid communication with the space surrounding said resiliently deformable members.

Preferably, the at least one non-porous face of the envelope includes at least one access conduit through the surface thereof and communicating with the envelope interior to provide a means of fluid evacuation from the envelope.

Preferably, the sealing means is a non-return valve associated with the access conduit to prevent return of envelope evacuated fluid therethrough.

In practice, the fluid is preferably gas, typically air.

Advantageously, it has been found that fluid evacuation from around the resiliently deformable beads is found to cause the beads to compress together providing a rigid or semi-rigid cover. By maintaining the sheet envelope, where the beads are located, in a fluid evacuated state, the rigidity of the cover is also maintained providing a protective sheet for any external object around which it is located. This is particularly important for fragile objects.

A vacuum pump may be provided and be associated with the non-return valve. The pump is typically a mechanical pump but alternative fluid evacuation means is also envisaged such as a chemical trigger which causes the air/fluid in the sheet envelope to be consumed by a chemical reaction, thus providing the required evacuation of the sheet envelope.

The sealing means may at least be partially provided by a closure member at the opening to the inside of the package so that the package is retained in a fluid evacuated state until the package closure member is opened by the user at which point the envelopes will re-inflate with air from the surrounding environment.

Preferably, the porous inner lining is in the form of a bag. Alternatively, the lining may be in the form of a sleeve.

The device may be in the form of a packaging bag and may be formed in any suitable manner. By way of example only, the bag may comprise two or more sheets or may comprise a single sheet folded into a bag with a porous face forming the interior of the bag and a non-porous face forming the exterior thereof or the bag exterior and interior may be formed from a single sheet or each may be formed from a single sheet. Typically, the resiliently deformable members are sandwiched between the inner lining and the non-porous outer envelope.

Preferably, in the said partially rigid state of the said members, the members collectively form a barrier to protect an object located within or adjacent to the said device. Preferably, in the said partially rigid state the members are urged into contact with adjacent members and remain in said contact during the fluid evacuated stage.

Preferably, the members are beads such as polymeric beads. The beads or members may be air filled to form, for example, balls, bubbles or the like. The beads or members may be any suitable shape and need not be spherical but spherical or hemi-spherical beads are generally preferred.

The fluid sealable envelope may be in the form of a package in which an object to be protected can be located via a suitable sealable opening. Preferably, the said object is located within the porous inner lining which is itself located in the fluid sealable envelope. Preferably, the opening(s) to the fluid sealable envelope and the inner lining are coincidental but need not necessarily be so. After location of the object, the opening can be resealed and the fluid, generally air, evacuated. The effect of fluid evacuation is to force the said members into closer proximity around the object and at the same time to thereby increase rigidity to form a protective barrier around the object.

According to a second aspect of the present invention, there is provided a method of protecting an object comprising
(i) locating an object to be protected in a protective device, the protective device comprising a fluid sealable envelope of a non-porous material, the said envelope having a plurality of resiliently deformable members located therein, the envelope being designed to have a fluid evacuated state in which the said plurality of resiliently deformable members is operable to form into an at least partially rigid state within the envelope and sealing means to retain the envelope in the said fluid evacuated state, the device including a porous inner lining, wherein the lining comprises a porous inner envelope which with the space surrounding said resiliently deformable members;
(ii) evacuating fluid from the device to form an at least partially rigid barrier with the said members located around the said object;
(iii) retaining the device in the said fluid evacuated state to thereby maintain the object in a protected state.

Preferably, the sealing means is a non-return valve associated with the access conduit to prevent return of envelope evacuated fluid therethrough.

The access conduit may extend only into the envelope or it may extend into the inner space defined by the inner lining. Advantageously, by exiting the access conduit into the space defined by the inner lining the beads may form a tighter fit around the object to be protected in the said inner lining.

In practice, the fluid is preferably gas, typically air.

The inner linings are porous so as not to hinder fluid evacuation from around an object located therein and the formation of the rigid barrier therearound.

Preferably, the process of fluid evacuation takes place after location of the object in the envelope or after wrapping of an object with the envelope. This causes a close fit of the resiliently deformable members around the object. Alternatively, it provides increased bending/twisting strength in the envelope compared with existing cushioned supports.

Optionally, the members may be concentrated in the centre area of the envelope against which an object to be protected will be located, or in any particular area where a greater member density is required. The resiliently deformable members may comprise a suitable foamed plastic such as polystyrene, polypropylene or polyvinyl chloride. The outer material may also be a suitable polymer such as polypropylene or nylon.

Additionally, in such embodiments, the package may also include further sealing means in the form of an access conduit and non return valve in the exterior wall of the package. However, this access conduit is not essential as fluid may be evacuated by vacuum pump during packaging and then the access conduit heat sealed so that it is not present in the final package product.

The accessable interior of such package bags may be sealed off, in use, via a plastic sealing strip/zip or other suitable sealing device, which can be reopened by the user to gain access to the object located in the package. In some cases, the package may be heat sealed in such manner as to require tearing or cutting to allow opening and re-inflation. As mentioned above, in some industrial applications, the fluid evacuation may take place followed by sealing of the access conduit. In this regard, the invention extends to products which have had the said access conduit sealed. This type of design may apply to single use products which may, thereafter, be returned to the manufacturer or discarded for recycling.

Preferably, the process of fluid evacuation takes place after location of the object in a package made from the sheet or after wrapping of an object with a sheet. This causes a close fit of the resiliently deformable beads around the object. Alternatively, it provides increased bending/twisting strength in the sheet compared with existing cushioned supports.

Preferably, the resiliently deformable members or beads form an increased rigidity layer, in use. Preferably, sufficient members or beads are provided to allow surface coverage of the target object. Typically, the member or bead barrier may be up to seven bead layers deep, preferably, up to 5 bead layers deep, more preferably up to 3 bead layers deep. Obviously, the depth of the barrier may vary across the sheet and the reference to bead layers above is a reference to the average bead layer depth in contact with the target object.

In one embodiment a pump may be integral with the access conduit so that the envelope may be evacuated by the user activating the pump manually. Alternatively, the pump may be a separate device for mating with the access conduit.

The evacuation can be carried out manually by the user or may be automated for certain applications, for instance, those involving mass packaging.

Preferably, the resiliently deformable member beads are made from thermally insulating material. As a result, the invention has applications where heat insulation of an object is important. For instance, food or biological samples could be transported using the invention to heat insulate aswell as protect the samples in transit and storage.

For applications involving electronic components, the invention may also comprise an anti-static sleeve for object location therein or the invention may be made from a material with antistatic properties.

Preferably, to avoid settling of the members or beads, they are apportioned into partitioned sections or otherwise apportioned to thereby provide a predetermined distribution thereof across the sheet, preferably, a substantially even distribution thereof across the envelope.

Preferably, the said partitions allow fluid communication between the said sections to thereby allow the required fluid evacuation of the whole envelope from any one section.

Optionally, the partitions concentrate beads in the centre area of the envelope, against which an object to be protected will be located, or in any particular area where a greater bead density is required.

Preferably, the beads are free moving within the envelope or partitioned section, as the case may be, prior to fluid evacuation.

The pads may comprise a suitable foamed plastic such as polystyrene or polyvinylchloride. The outer material may also be a suitable polymer such as polypropylene or nylon.

Advantageously, a product in accordance with the invention provides cushioning as well as rigidity.

Preferably, the members or beads are 1-20mm in width, more preferably 2-15mm, most preferably 3-7mm. Typically, the members or beads have a volume of 0.001-5 × 10⁶ mm³, more preferably, 0.1-5 × 10⁴ mm³, most preferably 1-5 × 10³ mm³.

Preferably, the envelope material is made from a substantially tear resistant material such as, for example, polypropylene.

The invention will now be described, by way of example, with reference to the accompanying drawings in which:-
Figure 1 shows a perspective view of a package in accordance with the present invention prior to assembly;
Figure 2 shows a built-in pump in the wall of a package in accordance with the present invention;
Figure 3 shows an external pump applied to a package in accordance with the present invention;
Figure 4 shows a valve extending into the space defined by the inner porous lining.

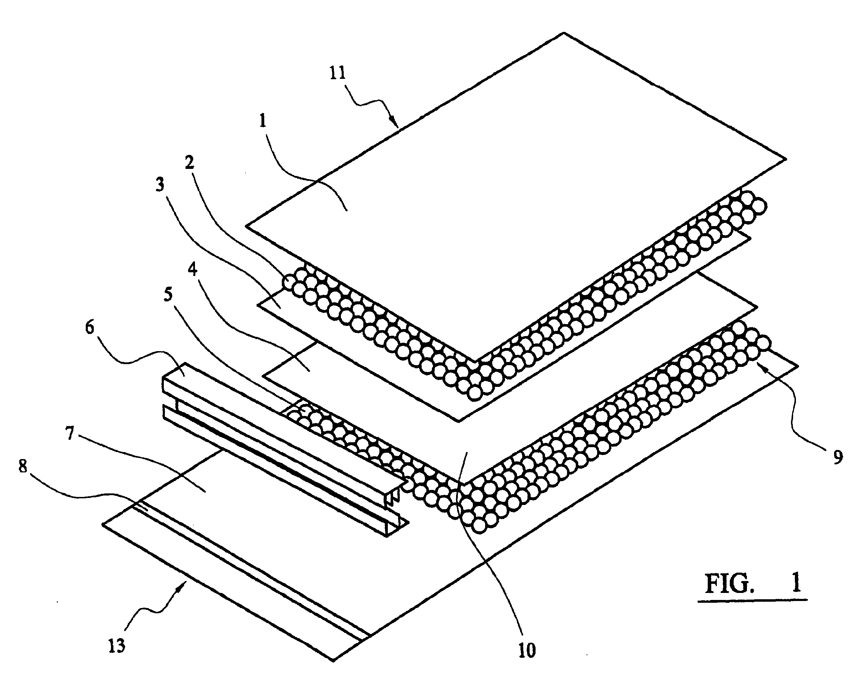

Referring to figure 1, a package incorporates two facing sheets 11, 9 each having a non-porous outer layer of non-porous polypropylene 1, 7 and a superimposed inner layer of perforated polypropylene 3, 4. Sandwiched between the said inner 3 and outer 1 layers is a layer of blown polystyrene beads 2, 5. In use, the inner and outer layers are heat sealed along their mutually opposed edges to provide an envelope for the layer of polystyrene beads 2. One of the outer layers 7 has an overlapping flap portion 8 at one end thereof which extends the back of the package 9 and may be folded over the front of the package, in use, to provide a closure flap. To produce a suitable package bag, the two envelope sheets 11, 9 are, in turn, heat sealed along opposed edges along three sides to thereby provide a bag which is open at one end 10. The open end 10 of the bag is provided with a sealing strip 6 to substantially fluid seal the open end, in use. The sealing strip is of conventional construction and comprises a pair of mutually co-operating inner jaws each with a flat outer strip which is heat sealed along the open section to the corresponding inner surface of the perforated strip 3, 4. By closing the sealing strip 6 the bag interior provided between the perforated inner sheets 3 and 4 is sealed so that a vacuum may be applied via one of the outer sheets 1, 7 to thereby evacuate air from the respective envelope and the bag interior, causing the bag sides to become rigid and thereby protecting an object located inside the bag.

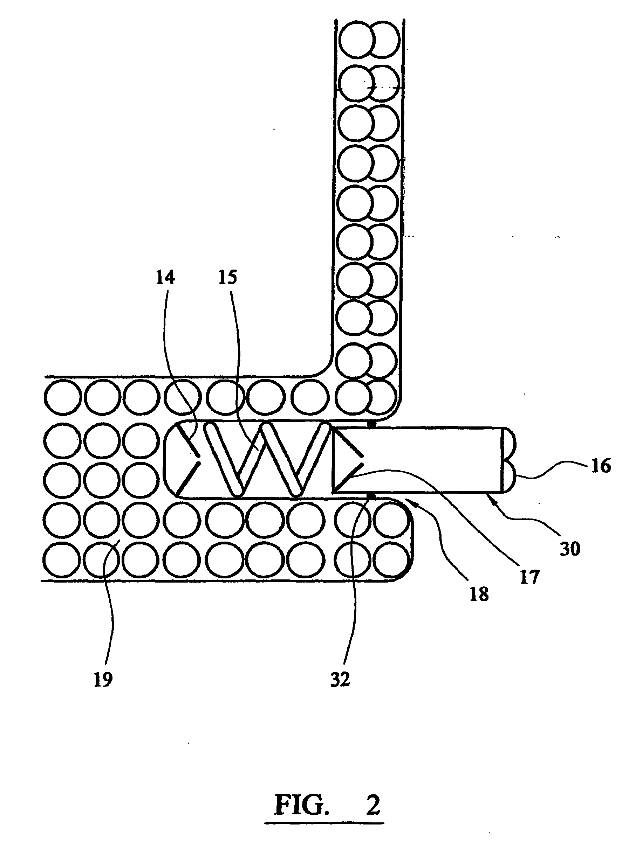

Referring to figure 2, a suitable pump 30 for fluid extraction from the bead envelope 19 is shown. The pump includes a thumb grip 16 which sealingly engages via sealing ring 32 with a close fitting cavity 18 located in the side of the bag and extending into the bead envelope, via a compression spring 15. The thumb grip is fitted at its inner end with a one way valve 17 and the innermost end of the cavity is similarly fitted with a one way valve 14. Accordingly, in use, the thumb grip 16 is pressed into the cavity 18 thereby forcing the air in the cavity to be expelled via the one way valve 17. Release of the thumb grip causes air to be drawn into the cavity 18 as the compression spring 15 forces the thumb grip 16 to its outermost position. At the limit of its compression, the thumb grip 16 has a catch (not shown) which allows the thumb grip to remain compressed into the cavity and hence conveniently stored in the side of the bag, during use.

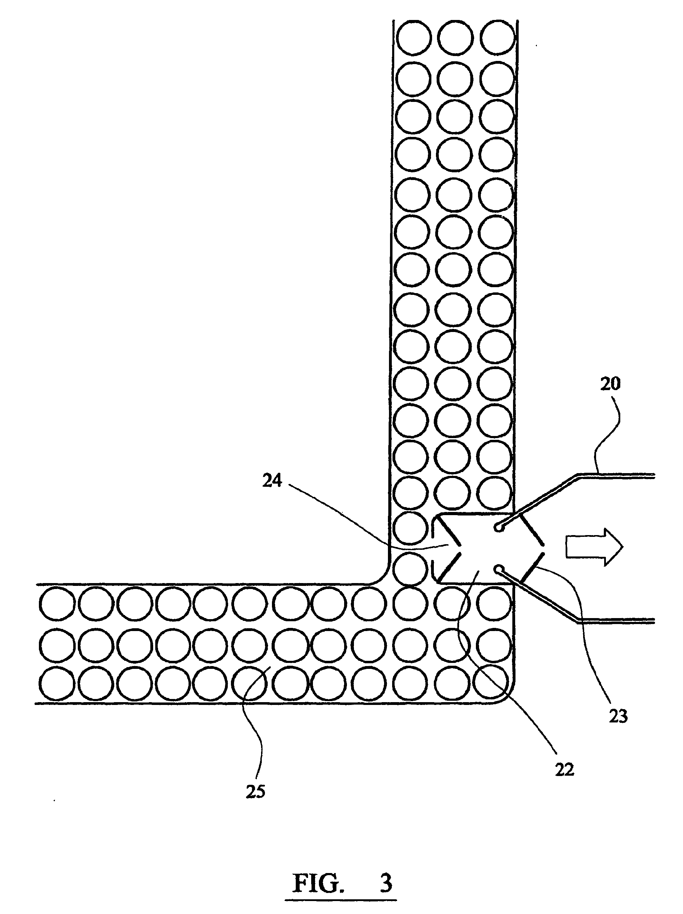

An alternative mechanism for extracting the fluid from the sheet envelope is shown in figure 3. An external air pump (not shown in entirety) is fitted with a nozzle 20 which is tapered at its outer end to form a sealing engagement with the outer end of a valve cavity 22 in the side of the bag. The cavity 22 is fitted at its innermost end with a one way valve 24 of the type as previously described with respect to figure 2 and, similarly, a one way valve is fitted to the interior end of the tapered nozzle so that air can be extracted from the envelope between the inner and outer sheets of the bag via the said pair of one way valves. Removal of the air pump from the bag is possible after a suitable level of air extraction has been effected via the one way valve 24. The one way valve 24, although allowing evacuation of the envelope 25 prevents ingress of air during use.

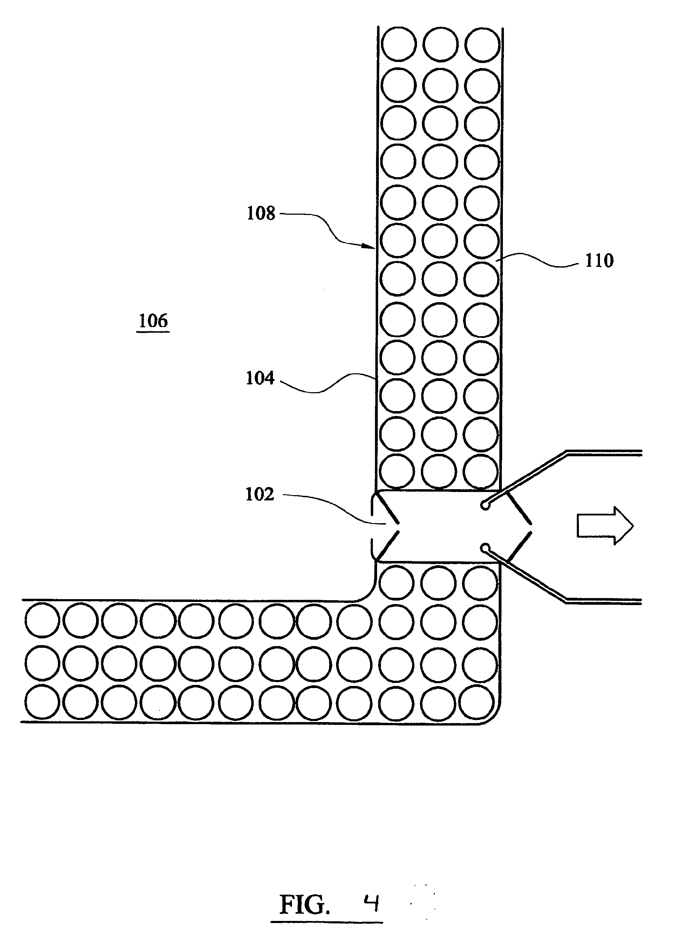

Referring to figure 4, a further embodiment shows a similar arrangement to that shown in figure 3 but with the valve 102 extending through the porous inner wall 104 of the bag to have direct access to the interior space 106 defined by the inner bag 108. In use, the air is evacuated from the said interior space so that the inner bag 108 collapses around an object located therein. Simultaneously, the air is evacuated from the cavity 110 via the pores of the inner wall 104 so that a rigid layer forms around the collapsed inner wall. The porous inner wall is made from a material suitable for use in the evacuation process.

For the avoidance of doubt, the porosity of the inner lining is sufficient to allow air or other fluid to transfer through the wall but is not sufficient to allow the resilient members or the internal object located in the bag to transfer through the wall.

## Claims

1. A protective device comprising a fluid sealable envelope (1, 7) of a non-porous material, the said envelope (1, 7) having a plurality of resiliently deformable members (2, 5) located therein, the envelope (1, 7) being designed to have a fluid evacuated state in which the said plurality of resiliently deformable members (2, 5) is operable to form into an at least partially rigid state within the envelope (1, 7) and sealing means (6) to retain the envelope (1, 7) in the said fluid evacuated state wherein the device includes a porous inner lining (3, 4, 104) designed to accommodate an object to be protected and wherein the lining (3, 4, 104) comprises a porous inner envelope, (3, 4, 104) **characterised in that** the envelope is in fluid communication with the space surrounding said resiliently deformable members (2, 5).

2. A device as claimed in claim 1, wherein the porous inner lining (3, 4, 104) is in the form of a bag (3, 4, 104).

3. A device as claimed in claim 1, wherein the porous inner lining (3, 4, 104) in the form of a sleeve (3, 4, 104).

4. A device as claimed in any preceding claim, wherein the resiliently deformable members (2, 5) are sandwiched between the porous inner lining (3, 4, 104) and the non-porous outer envelope (1, 7).

5. A protective device according to any preceding claim, wherein the non-porous fluid sealable envelope (1, 7) includes at least one access conduit (18) providing fluid communication from the exterior environment to the envelope interior to thereby provide a means of fluid evacuation from the envelope (1, 7).

6. A protective device according to any preceding claim, wherein the sealing means is a non-return valve (14) associated with the access conduit (18) to prevent return of envelope evacuated fluid therethrough.

7. A protective device according to any preceding claim, wherein a vacuum pump (30) is provided and associated with the sealing means.

8. A protective device according to any preceding claim, in the form of a package and wherein the sealing is at least partially provided by a closure member (24) at the opening to the inside of the package so that the package is retained in a fluid evacuated state until the package closure member (24) is opened by the user at which point the envelopes will re-inflate with fluid from the surrounding environment.

9. A device as claimed in any preceding claim, wherein the access conduit (18) extends into the inner space defined by the inner lining (3, 4, 104) to thereby provide means of priority evacuation of the said inner space.

10. A method of protecting an object comprising
(i) locating an object to be protected in a protective device, the protective device comprising a fluid sealable envelope (1, 7) of a non-porous material, the said envelope (1, 7) having a plurality of resiliently deformable members (2, 5) located therein, the envelope (1, 7) being designed to have a fluid evacuated state in which the said plurality of resiliently deformable members (2, 5) is operable to form into an at least partially rigid state within the envelope (1, 7) and sealing means (6) to retain the envelope (1, 7) in the said fluid evacuated state, the device including a porous inner lining designed to accommodate the object to be protected;
(ii) evacuating fluid from the device to form an at least partially rigid barrier with the said members (2, 5) located around the said object;
(iii) retaining the device in the said fluid evacuated state to thereby maintain the object in a protected state;
**characterised in that** the lining (3, 4, 104) comprises a porous inner envelope (1,7) which is in fluid communication with the space surrounding said resiliently deformable members (2, 5).

## Patentansprüche

1. Schutzvorrichtung, umfassend eine fluidversiegelbare Hülle (1, 7) aus einem nicht porösen Material, wobei die Hülle (1, 7) eine Mehrzahl von darin angeordneten federnd verformbaren Elementen (2, 5) aufweist, wobei die Hülle (1, 7) dazu ausgelegt ist, einen fluidevakuierten Zustand zu haben, in welchem die Mehrzahl von federnd verformbaren Elementen (2, 5) betriebsmäßig eine Form mit einem zumindest teilweise starren Zustand innerhalb der Hülle (1, 7) einnimmt, sowie eine Versiegelungseinrichtung (6), um die Hülle (1, 7) in dem fluidevakuierten Zustand zu halten, wobei die Vorrichtung ein poröses Innenfutter (3, 4, 104) umfaßt, das dazu ausgelegt ist, ein zu schützendes Objekt aufzunehmen, und wobei das Futter (3, 4, 104) eine poröse Innenhülle (3, 4, 104) umfaßt, **dadurch gekennzeichnet, dass** die Hülle in Fluidverbindung mit dem Raum ist, der die federnd verformbaren Elemente (2, 5) umgibt.

2. Vorrichtung nach Anspruch 1, wobei das poröse Innenfutter (3, 4, 104) die Form einer Tasche (3, 4, 104) aufweist.

3. Vorrichtung nach Anspruch 1, wobei das poröse Innenfutter (3, 4, 104) die Form einer Hülse (3, 4, 104) aufweist.

4. Vorrichtung nach einem der vorhergehenden Ansprüche, wobei die federnd verformbaren Elemente (2, 5) sandwichartig zwischen dem porösen Innenfutter (3, 4, 104) und der nicht porösen äußeren Hülle (1, 7) eingeschlossen sind.

5. Schutzvorrichtung nach einem der vorhergehenden Ansprüche, wobei die nicht poröse fluidversiegelbare Hülle (1, 7) mindestens eine Zugangsleitung (18) umfaßt, die eine Fluidverbindung von der äußeren Umgebung zum Inneren der Hülle bereitstellt, um hierdurch ein Mittel zur Fluidevakuierung aus der Hülle (1, 7) bereitzustellen.

6. Schutzvorrichtung nach einem der vorhergehenden Ansprüche, wobei die Versiegelungseinrichtung ein Rückschlagventil (14) ist, das der Zugangsleitung (18) zugeordnet ist, um eine Rückkehr von aus der Hülle evakuiertem Fluid durch sie hindurch zu verhindern.

7. Schutzvorrichtung nach einem der vorhergehenden Ansprüche, wobei eine Vakuumpumpe (30) bereitgestellt und der Versiegelungseinrichtung zugeordnet ist.

8. Schutzvorrichtung nach einem der vorhergehenden Ansprüche, in der Form eines Pakets, wobei die Versiegelung wenigstens teilweise durch ein Schließelement (24) an der Öffnung zur Innenseite des Pakets bereitgestellt ist, so dass das Paket in einem fluidevakuierten Zustand gehalten wird, bis das Paketschließelement (24) durch den Benutzer geöffnet wird, zu welchem Zeitpunkt die Hülle durch Fluid aus der Umgebung wieder aufgeblasen wird.

9. Vorrichtung nach einem der vorhergehenden Ansprüche, wobei sich die Zugangsleitung (18) in den Innenraum hinein erstreckt, der durch das Innenfutter (3, 4, 104) definiert ist, um hierdurch eine Einrichtung zur prioritären Evakuierung des Innenraums bereitzustellen.

10. Verfahren zum Schützen eines Objekts, umfassend die folgenden Schritte:
(i) Anordnen eines zu schützenden Objekts in einer Schutzvorrichtung, wobei die Schutzvorrichtung eine fluidversiegelbare Hülle (1, 7) aus einem nicht porösen Material umfaßt, wobei die Hülle (1, 7) eine Mehrzahl von darin angeordneten federnd verformbaren Elementen (2, 5) aufweist, wobei die Hülle (1, 7) dazu ausgelegt ist, einen fluidevakuierten Zustand zu haben, in welchem die Mehrzahl von federnd verformbaren Elementen (2, 5) betriebsmäßig eine Form mit einem wenigstens teilweise starren Zustand innerhalb der Hülle (1, 7) einnimmt, sowie eine Versiegelungseinrichtung (6), um die Hülle (1, 7) in dem fluidevakuierten Zustand zu halten, wobei die Vorrichtung ein poröses Innenfutter umfaßt, das dazu ausgelegt ist, das zu schützende Objekt aufzunehmen;
(ii) Evakuieren von Fluid aus der Vorrichtung, um eine wenigstens teilweise starre Barriere mit den um das Objekt herum angeordneten Elementen (2, 5) zu bilden;
(iii) Halten der Vorrichtung in dem fluidevakuierten Zustand, um hierdurch das Objekt in einem geschützten Zustand zu halten;
**dadurch gekennzeichnet, dass** das Futter (3, 4, 104) eine poröse Innenhülle (1, 7) umfaßt, die in Fluidverbindung mit dem Raum ist, der die federnd verformbaren Elemente (2, 5) umgibt.

## Revendications

1. Dispositif de protection comprenant une enveloppe scellable au fluide (1, 7) en un matériau non poreux, ladite enveloppe (1, 7) ayant une pluralité d'éléments déformables de façon élastique (2, 5) positionnés à l'intérieur, l'enveloppe (1, 7) étant conçue pour avoir un état de fluide évacué dans lequel ladite pluralité d'éléments déformables de façon élastique (2, 5) sont utilisables pour prendre au moins un état partiellement rigide à l'intérieur de l'enveloppe (1, 7) et des moyens d'étanchéité (6) pour maintenir l'enveloppe (1, 7) dans ledit état de fluide évacué dans lequel le dispositif inclut un revêtement interne poreux (3, 4, 104) conçu pour loger un objet devant être protégé et dans lequel le revêtement (3, 4, 104) comprend une enveloppe interne poreuse (3, 4, 104), l'enveloppe étant en communication fluide avec l'espace entourant lesdits éléments déformables de façon élastique (2, 5).

2. Dispositif selon la revendication 1, dans lequel le revêtement interne poreux (3, 4, 104) est sous la forme d'un sac (3, 4, 104).

3. Dispositif selon la revendication 1, dans lequel le revêtement interne poreux (3, 4, 104) est sous la forme d'un manchon (3, 4, 104).

4. Dispositif selon l'une quelconque des revendications précédentes, dans lequel les éléments déformables de façon élastique (2, 5) sont pris en sandwich entre le revêtement interne poreux (3, 4, 104) et l'enveloppe externe non poreuse (1, 7).

5. Dispositif de protection selon l'une quelconque des revendications précédentes, dans lequel l'enveloppe scellable au fluide non poreuse (1, 7) comprend au moins un conduit d'accès (18) assurant une communication fluide depuis l'environnement extérieur vers l'intérieur de l'enveloppe pour fournir ainsi des moyens d'évacuation de fluide à partir de l'enveloppe (1, 7).

6. Dispositif de protection selon l'une quelconque des revendications précédentes, dans lequel les moyens d'étanchéité sont un clapet anti-retour (14) associé au conduit d'accès (18) pour empêcher le retour du fluide évacué de l'enveloppe à travers lui.

7. Dispositif de protection selon l'une quelconque des revendications précédentes, dans lequel une pompe à vide (30) est prévue et associée aux moyens d'étanchéité.

8. Dispositif de protection selon l'une quelconque des revendications précédentes, sous la forme d'un boîtier et dans lequel l'étanchéité est au moins partiellement assurée par un élément de fermeture (24) au niveau de l'ouverture vers l'intérieur du boîtier de sorte que le boîtier soit maintenu dans un état de fluide évacué jusqu'à ce que l'élément de fermeture de boîtier (24) soit ouvert par l'utilisateur, auquel moment les enveloppes se regonfleront avec du fluide provenant de l'environnement voisin.

9. Dispositif selon l'une quelconque des revendications précédentes, dans lequel le conduit d'accès (18) s'étend dans l'espace interne défini par le revêtement interne (3, 4, 104) pour fournir ainsi des moyens d'évacuation prioritaire dudit espace interne.

10. Procédé de protection d'un objet consistant à
(i) positionner un objet devant être protégé dans un dispositif de protection, le dispositif de protection comprenant une enveloppe scellable au fluide (1, 7) en un matériau non poreux, ladite enveloppe (1, 7) ayant une pluralité d'éléments déformables de façon élastique (2, 5) positionnés à l'intérieur, l'enveloppe (1, 7) étant conçue pour avoir un état de fluide évacué dans lequel ladite pluralité d'éléments déformables de façon élastique (2, 5) sont utilisables pour prendre au moins un état partiellement rigide à l'intérieur de l'enveloppe (1, 7) et des moyens d'étanchéité (6) pour maintenir l'enveloppe (1, 7) dans ledit état de fluide évacué, le dispositif comprenant un revêtement interne poreux (3, 4, 104) conçu pour loger l'objet devant être protégé ;
(ii) évacuer le fluide du dispositif pour former une barrière au moins partiellement rigide avec lesdits éléments (2, 5) positionnés autour dudit objet ;
(iii) maintenir le dispositif dans ledit état de fluide évacué pour maintenir ainsi l'objet dans un état protégé ;
**caractérisé en ce que** le revêtement (3, 4, 104) comprenant une enveloppe interne poreuse (1, 7) qui est en communication fluide avec l'espace entourant lesdits éléments déformables de façon élastique (2, 5).
